# EUROPÄISCHE PATENTSCHRIFT

(11) **EP 1 646 420 B1**
(45) Veröffentlichungstag und Bekanntmachung des Hinweises auf die Patenterteilung: **13.06.2007**
(21) Anmeldenummer: 04741197.0
(22) Anmeldetag: 21.07.2004
(51) Int. Cl.: A61M 25/06, A61M 5/158

(54) **Einstechvorrichtung zum einstechen einer Injektionsnadel**
Insertion device for inserting an injection needle
Dispositif permettant de planter une aiguille d'injection

(30) Priorität: 21.07.2003 DE 10333118
(43) Veröffentlichungstag der Anmeldung: 19.04.2006
(73) Patentinhaber: Disetronic Licensing AG, 3401 Burgdorf (CH)
(72) Erfinder: BUETIKOFER, Markus, CH-3110 Münsingen (CH); SCHEURER, Simon, CH-3006 Bern (CH); WYSS, Martin, CH-3510 Konolfingen (CH)
(74) Vertreter: Schalch, Rainer
(86) Internationale Anmeldenummer: PCT/EP2004/008156
(87) Internationale Veröffentlichungsnummer: WO 2005/009525

(56) Entgegenhaltungen:
- EP-A- 0 734 739
- EP-A- 0 747 084
- US-A- 4 950 252
- US-A- 6 090 068

## Beschreibung

Die Erfindung betrifft eine Einstechvorrichtung zum Einstechen einer Injektionsnadel in ein Gewebe. Die Einstechvorrichtung kann insbesondere Teil eines Katheterkopfes sein, wobei der Katheterkopf z. B. ein Teil eines Infusionssets bilden kann.

Aus dem Stand der Technik sind viele verschiedenartige Einstechvorrichtungen zum Einstechen einer Injektionsnadel in ein Gewebe bekannt. Neuere Einstechvorrichtungen umfassen neben einem Nadelhalter, von dem die Injektionsnadel abragt, eine Schutzvorrichtung zum Schutz der Injektionsnadel nach dem Herausziehen aus dem Gewebe. Die Schutzvorrichtung dient dazu, den Anwender der Einstechvorrichtung vor Stichverletzungen zu schützen, da die Injektionsnadel nach dem Herausziehen aus dem Gewebe frei zugänglich ist. Solche Einstechvorrichtungen werden bei verschiedenen medizinischen oder therapeutischen Vorrichtungen verwendet, bei welchen ein fluider Wirkstoff oder eine andere Flüssigkeit in ein Körpergewebe eingebracht werden muss.

Die vorliegende Erfindung geht insbesondere von einer Einstechvorrichtung für einen Katheterkopf z. B. für ein Infusionsset aus. Der Katheterkopf dient als Port zum Einleiten eines flüssigen Wirkstoffs, wie z. B. Insulin oder Wachstumshormone, in das Körpergewebe. Hierfür wird der Katheterkopf für eine längere Zeit auf der Oberfläche des Körpergewebes angebracht. Von einer Unterseite des Katheterkopfes ragt eine flexible Kanüle in das Körpergewebe hinein. Durch ihre Flexibilität kann die Kanüle den Bewegungen und Veränderungen des Körpergewebes folgen und verursacht keine Unannehmlichkeiten für den Anwender des Katheterkopfes. Über der Gewebeoberfläche führt von dem Katheterkopf ein Katheter zu einem Fluidbehälter mit dem flüssigen Wirkstoff. Der Katheter steht mit der Kanüle in einer Flüssigkeitsverbindung. Zum Einsetzen der flexiblen Kanüle in das Körpergewebe kann eine steife Injektionsnadel verwendet werden, die z. B. durch die Kanüle geführt wird, so dass diese fest an der Injektionsnadel anliegt, und die mit ihrer Einstechspitze über die Kanüle hinausragt, Nach dem Einführen der Kanüle in das Gewebe wird die steife Injektionsnadel aus der Kanüle gezogen, wobei die Kanüle in dem Gewebe verbleibt. Die Injektionsnadel bildet demnach eine Einstechhilfe für die flexible Kanüle.

Eine Einstechvonichtung mit einer Injektionsnadel als Stechhilfe für einen Katheterkopf wie oben beschrieben, ist z. B. in der WO 00/03757 beschrieben. Die Einstechvorrichtung weist ein Gehäuse auf, von dem eine Injektionsnadel derart abragt, dass sie durch einen Grundkörper des Katheterkopfes hindurch reicht und, durch die flexible Kanüle des Katheterkopfes verläuft. An einer Seite des Gehäuses der Einstechvorrichtung ist eine Schutzvonichtung in Form einer gegenüber dem Gehäuse schwenkbaren Hülsenkappe angeordnet, die zu einer Seite in Richtung der Nadel offen ist. In einem ersten Zustand zum Einstechen der Injektionsnadel ragt die Hülsenkappe senkrecht zur Nadelachse von dem Gehäuse ab. Nach dem Einstechen der Injektionsnadel, bzw. der flexiblen Kanüle, wird die Einstechvorrichtung von dem Grundkörper des Katheterkopfes abgenommen, wobei die Injektionsnadel aus dem Gewebe, bzw. der flexiblen Kanüle herausgezogen wird. Nach dem Herausziehen der Einstechvorrichtung kann dann die Hülsenkappe relativ zum Gehäuse über die Injektionsnadel geschwenkt werden. Dabei wird die Injektionsnadel in Schwenkrichtung der Hülsenkappe zur Seite gebogen und von dieser teilweise umgeben. Auf der Seite des Gehäuses, die der Hülsenkappe gegenüberliegt, sind Rasteinrichtungen vorgesehen, in welche die Hülsenkappe nach ihrem Schwenken einrastet, so dass sie nicht durch die Rückstellkraft der verbogenen Nadel zurückgeschwenkt wird. Die Injektionsnadel liegt jedoch nach dem Herausziehen aus dem Gewebe bzw. dem Grundkörper des Katheterkopfes zunächst frei, wodurch eine, Verletzungsgefahr für den Anwender entsteht Ferner muss die Hülsenkappe manuell über die Injektionsnadel geführt werden, wodurch ein zusätzliches Verletzungsrisiko entstehen kann, da diverse Handgriffe in der Nähe der Injektionsnadel durchgeführt werden müssen.

US 4 950 252 offenbart eine Einstechvorrichtung mit den Merkmalen im Oberbegriff des unabhängigen Anspruchs 1.

Es ist eine Aufgabe der vorliegenden Erfindung, eine Einstechvorrichtung zum Einstechen einer Injektionsnadel in ein Gewebe zu schaffen, bei der nach dem Herausziehen der Injektionsnadel aus einem Gewebe kein Verletzungsrisiko entsteht, bei der eine Schutzvorrichtung zum Schutz der Injektionsnadel auf einfache Weise um die Injektionsnadel angeordnet werden kann, bei der die Injektionsnadel während dem Herausziehen zu jeder Zeit geschützt ist und für die keine zusätzlichen Handgriffe zum Schutz der Injektionsnadel nach dem Abnehmen der Einstechvorrichtung von einer Oberfläche erforderlich sind.

Ferner ist es eine Aufgabe der vorliegenden Erfindung, einen Katheterkopf für medizinische und pharmazeutische Anwendungen zu schaffen, bei dem kein Verletzungsrisiko durch eine Injektionsnadel als Einstechhilfe für eine flexible Kanüle entsteht, von dem die Einstechhilfe in einfacher Weise nach dem Einführen der flexiblen Kanüle abgenommen werden kann und der leicht auf einem Gewebe angebracht werden kann.

Die Aufgabe der Erfindung wird durch eine Einstechvorrichtung nach Anspruch 1 und durch einen Katheterkopf nach Anspruch 13 gelöst. Vorteilhafte Ausgestaltungen der Erfindung gehen aus den Unteransprüchen hervor.

Demnach umfasst eine Einstechvorrichtung nach der vorliegenden Erfindung zum Einstechen einer Injektionsnadel in ein Gewebe einen Nadelhalter, von dem die Injektionsnadel abragt, und eine Schutzvorrichtung zum Schutz der Injektionsnadel beim Herausziehen aus dem Gewebe. Der Nadelhalter ist gegenüber der Schutzvorrichtung entlang der Nadelachse verschiebbar gelagert. Hierfür können der Nadelhalter und die Schutzvorrichtung hülsenartig ausgebildet sein, so dass sie ineinander geschoben werden können. Der Nadelhalter kann dabei außen über die Schutzvorrichtung oder ins Innere der Schutzvorrichtung geschoben werden. Zum Einstechen der Injektionsnadel ist der Nadelhalter in einer mit der Schutzvorrichtung zusammengeschobenen Position. Die Injektionsnadel steht dabei über die Schutzvorrichtung hervor, so dass in das Gewebe eingestochen werden kann. In dieser Position kann die Injektionsnadel durch eine Schutzkappe vor Zugriff geschützt sein, die kurz vor dem Einstechen abgenommen wird. Beim Auseinanderziehen von Nadelhalter und Schutzvorrichtung wird der Nadelhalter gegenüber der Schutzvorrichtung entlang der Nadelachse in eine ausgezogene Position verschoben, in der die Spitze der Injektionsnadel von der Schutzvorrichtung umgeben ist. Vorzugsweise steht die Schutzvorrichtung dabei gegenüber dem Gewebe fest. Die Injektionsnadel wird aus dem Gewebe direkt ins Innere der Schutzvorrichtung gezogen und liegt zu keinem Zeitpunkt frei. Um den Nadelhalter und die Schutzvorrichtung wenigstens in der ausgezogenen Position fest miteinander zu verrasten, ist an dem Nadelhalter und/oder der Schutzvorrichtung wenigstens eine Rasteinrichtung vorgesehen. Dadurch kann der Nadelhalter nach einem. Verrasten nicht zurück in eine zusammengeschobene Position fallen, in der die Injektionsnadel aus der Schutzvorrichtung herausragen könnte. Es ist dabei besonders vorteilhaft, wenn die wenigstens eine Rasteinrichtung bei einem Auseinanderziehen von Nadelhalter und Schutzvorrichtung ein erneutes Zusammenschieben nicht erst in der vollständig ausgezogenen Position blockierte sondern auch bereits nach einem teilweisen Auseinanderziehen, bei dem noch ein Teil der Injektionsnadel innerhalb des Gewebes liegt, ein Zurückschieben blockiert.

Nach einem weiteren Aspekt der vorliegenden Erfindung bildet eine erfindungsgemäße Einstechvorrichtung einen Teil eines Katheterkopfes für medizinische und pharmazeutische Anwendungen, wie er z. B. aus der Anmeldung DE 102 55 817.5 der Anmelderin bekannt ist. Ein solcher Katheterkopf weist einen Grundkörper mit einer von diesem abragenden flexiblen Kanüle auf. Die Einstechvorrichtung dient zum Einführen der flexiblen Kanüle in ein Gewebe. Die Einstechvorrichtung ist dabei wie oben beschrieben ausgebildet.

Bei einer bevorzugten Ausführungsform der Erfindung ist die Schutzvorrichtung der Einstechvorrichtung mit dem Grundkörper des Katheterkopfes verbunden, wobei der Grundkörper auf einer Oberfläche des Gewebes aufliegt. Grundsätzlich ist es denkbar, die Schutzvorrichtung mit dem Grundkörper lösbar zu verbinden. In der beschriebenen Ausführungsform ist dies jedoch nicht notwendig. Die Injektionsnadel ragt in der zusammengeschobenen Position von Nadelhalter- und Schutzvorrichtung, durch den Grundkörper hindurch. Vorzugsweise ragt die Injektionsnadel senkrecht zu einer Auflagefläche des Grundkörpers zur Auflage auf der Gewebeoberfläche aus diesem hervor. Zum Einführen der flexiblen Kanüle des Katheterkopfes ragt die Injektionsnadel durch die flexible Kanüle und ihre Spitze über diese hinaus. Nach dem Einstechen der Injektionsnadel, bzw. der flexiblen Kanüle, in das Gewebe wird der Nadelhalter gegenüber der Schutzvorrichtung entlang der Nadelachse verschoben und dabei die Injektionsnadel aus dem Gewebe bzw. der Kanüle gezogen. In der ausgezogenen Position des Nadelhalters ist die Injektionsnadel vollständig in die Schutzvorrichtung zurückgezogen.

Wird es gewünscht, dass die Einstechvorrichtung lösbar an dem Katheterkopf vorgesehen ist, wird die Einstechvorrichtung vorzugsweise derart an dem Katheterkopf angeordnet, dass die Einstechvorrichtung in der ausgezogenen Position des Nadelhalters in eine Richtung senkrecht zur Nadelachse, d. h .parallel zu einer Oberfläche des Gewebes, von dem Grundkörper abgezogen werden kann. Dadurch kann vermieden werden, dass durch das Abnehmen der Einstechvorrichtung von dem Katheterkopf der Katheterkopf von der Gewebeoberfläche abgehoben wird und die flexible Kanüle zumindest teilweise aus dem Gewebe gezogen wird. Grundsätzlich ist es jedoch auch möglich, die Einstechvorrichtung in einer Richtung parallel zur Nadelachse von dem Grundkörper abzunehmen.

Durch eine Einstechvorrichtung und einem Katheterkopf nach der vorliegenden Erfindung ist es möglich, eine Injektionsnadel aus einem Gewebe zu entfernen, ohne das dadurch ein Verletzungsrisiko für den Anwender entsteht. Die Injektionsnadel ist während dem Herausziehen aus dem Gewebe und in der herausgezogenen Position zu jeder Zeit verdeckt und unzugänglich. Durch die Rasteinrichtung zwischen Nadelhalter und Schutzvorrichtung ist es auch nach dem Herausziehen der Injektionsnadel in die herausgezogene Position des Nadelhalters gegenüber der Schutzvorrichtung nicht möglich, die Injektionsnadel frei zu legen. Der Nadelschutz wird in einfacher Weise bereits beim Herausziehen der Injektionsnadel aus dem Gewebe über der bzw. um die Injektionsnadel angeordnet und es ist kein zusätzlicher Arbeitsschritt notwendig, um die Schutzvorrichtung um die Injektionsnadel anzubringen. Die Einstechvorrichtung kann auf einfache Weise von dem Katheterkopf abgezogen werden, ohne das dabei die Gefahr besteht, dass der Katheterkopf von der Gewebeoberfläche angehoben wird. Dies ergibt sich daraus, dass die Bewegung zum Lösen der Einstechvorrichtung von dem Katheterkopf senkrecht zur Bewegung zum Herausziehen der Injektionsnadel bzw. zum Herausziehen des Nadelhalters aus der Schutzvorrichtung verläuft.

Bei einer bevorzugten Ausführungsform der Erfindung ist zwischen Schutzvorrichtung und Nadelhalter wenigstens eine Führung zum Führen des Nadelhalters beim Verschieben gegenüber der Schutzvorrichtung entlang der Nadelachse vorgesehen. Die Führung kann z. B. durch eine oder mehrere Führungsrillen an der Schutzvorrichtung gegeben sein, in die ein an dem Nadelhalter angeordneter Vorsprung eingreift. Natürlich können die Führungsrillen auch an dem Nadelhalter und entsprechend die Vorsprünge an der Schutzvorrichtung vorgesehen sein. Die Rasteinrichtung weist zum Verrasten des Nadelhalters mit der Schutzvorrichtung in der ausgezogenen Position des Nadelhalters wenigstens einen ersten Anschlag zum Blockieren des Nadelhalters in Richtung des Auseinanderziehens und wenigstens einen zweiten Anschlag zum Blockieren des Nadelhalters in Richtung des Zusammenschiebens auf. Beispielsweise kann ein erster Anschlag dadurch gebildet werden, dass ein Vorsprung an dem Nadelhalter gegen ein Widerlager an der Schutzvorrichtung stößt, wenn der Nadelhalter in der ausgezogenen Position ist. Natürlich kann der Vorsprung auch an der Schutzvorrichtung und das Widerlager an dem Nadelhalter vorgesehen sein. Ein solches Widerlager kann z. B. durch eine Stufe oder einen Vorsprung oder eine äußere Oberfläche des Nadelhalters oder der Schutzvorrichtung gegeben sein. Der zweite Anschlag zum Blockieren des Zusammenschiebens wird vorzugsweise an wenigstens einem blattfederartigen Element angeordnet. Der zweite Anschlag kann durch einen Vorsprung an dem blattfederartigen Element vorgesehen sein, der in der ausgezogenen Position des Nadelhalters gegen ein Widerlager stößt. Für die Rasteinrichtung können ein oder mehrere blattfederartige Elemente an dem Nadelhalter oder an der Schutzvorrichtung oder auch an beiden vorgesehen sein. Ein blattfederartiges Element wird z. B. durch einen lattenartigen Fortsatz an dem Nadelhalter oder der Schutzvorrichtung gebildet, der im wesentlichen parallel zur Nadelachse von dem Nadelhalter in Richtung der Schutzvorrichtung, bzw. von der Schutzvorrichtung in Richtung des Nadelhalters, abragt. Der Fortsatz ist fest an dem Nadelhalter bzw. der Schutzvorrichtung angeordnet. Vorzugsweise ist er einstückig mit dem Nadelhalter oder der Schutzvorrichtung gefertigt z. B. durch ein Spritzgussverfahren. Der Fortsatz ist jedoch gegenüber dem Nadelhalter oder der Schutzvorrichtung biegbar und kann dadurch unter Vorspannung gesetzt werden. Grundsätzlich kann das blattfederartige Element aber auch dadurch gebildet werden, dass es mit sich gegenüberliegenden Enden an dem Nadelhalter, bzw. der Schutzvorrichtung fest ist.

Es können natürlich auch andere Rasteinrichtungen statt blattfederartigen Elemente in Form von lattenähnlichen Fortsätzen mit den entsprechenden Anschlägen für eine Einstechvorrichtung der vorliegenden Erfindung verwendet werden, solange ein festes Verrasten in der ausgezogenen Position von Nadelhalter und Schutzvorrichtung möglich ist. Beispielsweise könnten Rastelemente verwendet werden, die durch Spiralfedern vorgespannt werden, oder elastische Gummielemente, die beim Auseinanderziehen des Nadelhalters und der Schutzvorrichtung komprimiert werden und in der ausgezogenen Position expandieren. Bei der Ausgestaltung der Rasteinrichtung ist lediglich darauf zu achten, dass der Rastmechanismus auf der Grundlage der Verschiebbarkeit des Nadelhalters 1 entlang der Längsachse der Injektionsnadel relativ zu der Schutzvorrichtung basiert, um ein Verrasten in Folge des Herausziehens der Nadel aus dem Gewebe zu ermöglichen.

Vorzugsweise sind der Nadelhalter und die Schutzvorrichtung quaderförmig mit einer breiten, bzw. langen und einer kurzen, bzw. schmalen Seite ausgebildet. Ein erster Anschlag zum Blockieren des Auseinanderziehens von Nadelhalter und Schutzeinrichtung wird aus zwei lattenartigen Fortsätzen gebildet, die jeweils an den sich gegenüberliegenden kurzen Seiten angeordnet sind. Der zweite Anschlag zum Blockieren des Zusammenschiebens von Nadelhalter und Schutzvorrichtung wird aus vier lattenähnlichen Fortsätzen gebildet, wobei jeweils zwei der Fortsätze an einer breiten Seiten angeordnet sind. Ein Zusammenschieben von Nadelhalter und Schutzvorrichtung wird dadurch durch vier symmetrisch angeordnete Angriffspunkte zwischen Nadelhalter und Schutzvorrichtung verhindert. Grundsätzlich ist es möglich, alle lattenartigen Fortsätze für den ersten und den zweiten . Anschlag entweder an dem Nadelhalter oder der Schutzvorrichtung vorzusehen. Es ist jedoch auch möglich, die Fortsätze für einen ersten Anschlag an dem Nadelhalter und die Fortsätze für einen zweiten Anschlag an der Schutzvorrichtung oder umgekehrt vorzusehen.

Bei einer Ausführungsform der Rasteinrichtung ist es möglich, dass die lattenartigen Fortsätze in der zusammengeschobenen Position von Nadelhalter und Schutzvorrichtung in einer vorgespannten Stellung sind und sich in der ausgezogenen Position in eine Blockierstellung entspannen. Bei einer anderen Ausführungsform ist es möglich, dass die lattenartigen Fortsätze in der eingeschobenen Position in einer entspannten Stellung sind und beim Auseinanderziehen von Nadelhalter und Schutzvorrichtung z. B. durch eine Rampe vorgespannt werden. Es ist vorteilhaft, wenn die Vorsprünge an den lattenartigen Fortsätzen durch das Auseinanderziehen in aufeinanderfolgende Widerlager z. B. innerhalb der Führung eingreifen und dadurch während des Auseinanderziehens bereits ein erneutes Zusammenschieben von Nadelhalter und Schutzvorrichtung verhindert wird. Das Verrasten des Nadelhalters mit der Schutzvorrichtung mittels der Rasteinrichtung kann demnach erfindungsgemäß durch ein Entspannen der lattenartigen Fortsätze oder durch ein Vorspannen der lattenartigen Fortsätze beim Auseinanderziehen erfolgen. Zum Blockieren des Nadelhalters gegenüber der Schutzvorrichtung in der ausgezogenen Position wirken die Vorsprünge an den lattenartigen Fortsätzen mit den jeweils gegenüberliegenden Widerlagern zusammen.

Es ist darauf zu achten, dass der Nadelhalter beim Einstechen der Injektionsnadel nicht schon allein durch den Widerstand den das Gewebe gegenüber der Injektionsnadel ausübt, gegenüber der Schutzvorrichtung verschoben wird. Falls die lattenartigen Fortsätze beim Herausziehen des Nadelhalters aus der Schutzvorrichtung vorgespannt werden, kann die Widerstandskraft der Fortsätze, die der Vorspannung entgegenwirkt, ausreichen, um ein Verschieben des Nadelhalters gegenüber der Schutzvorrichtung durch das Einstechen der Injektionsnadel zu verhindern. Es kann jedoch zusätzlich auch eine Verriegelungsvorrichtung zum Ver- bzw. Entriegeln des Nadelhalters mit der Schutzvorrichtung in der zusammengeschobenen Position an der Einstechvorrichtung vorgesehen sein. In der verriegelten Stellung ist der Nadelhalter gegenüber der Schutzvorrichtung nicht verschiebbar und kann daher auch beim Einstechen der Injektionsnadel in das Gewebe nicht aus Versehen aus der Schutzvorrichtung herausgeschoben werden. Nach dem Einstechen der Injektionsnadel kann die Verriegelungsvorrichtung entriegelt werden, so dass der Nadelhalter gegenüber der Schutzvorrichtung verschiebbar ist. Die Vemegelungsvonichtung kann vorteilhaft mit zwei Angriffspunkten für den Nadelhalter verbunden sein, so dass durch das Angreifen des Nadelhalters zum Herausziehen aus der Schutzvorrichtung gleichzeitig die Verriegelungsvorrichtung entriegelt wird. Dies kann z. B. durch Eindrücken von Angriffsflächen in den Nadelhalter erfolgen. Weiter kann die Verriegelungsvorrichtung auch mit der Rasteinrichtung gekoppelt sein. Hierfür können z. B. entsprechende Vorsprünge an den lattenartigen Fortsätzen vorgesehen sein, die bereits in der zusammengeschobenen Position gegen Widerlager stoßen und durch das Angreifen an den Angriffspunkten des Nadelhalters aus diesen Widerlagern gelöst werden.

Die vorliegende Erfindung wird in der Zeichnung an Ausführungsbeispielen beschrieben. Aus der Zeichnung und der zugehörigen Beschreibung offenbar werdende Merkmale der Erfindung sollen als zum Schutzumfang gehörend betrachtet werden. In der Zeichnung stellen dar:
- Figur 1: eine perspektivische Ansicht eines Nadelhalters eine Einstechvorrichtung in einer ersten Ausführungsform der vorliegenden Erfindung,
- Figur 2: eine perspektivische. Ansicht einer Einstechvorrichtung mit einem Nadelhalter nach Figur 1 in einer zusammengeschobenen Position von Nadelhalter und Schutzvorrichtung,
- Figur 3: eine perspektivische Ansicht einer Einstechvorrichtung nach Figur 2 in einer auseinandergezogenen Position,
- Figur 4: eine perspektivische Ansicht einer Schutzvorrichtung einer zweiten Ausführungsform der vorliegenden Erfindung,
- Figur 5: eine perspektivische Ansicht einer Einstechvorrichtung mit einer Schutzvorrichtung nach Figur 4 in einer zusammengeschobenen Position von Nadelhalter und Schutzvorrichtung und
- Figur 6: eine perspektivische Ansicht einer Einstechvorrichtung nach Figur 5 in einer auseinandergezogenen Position.

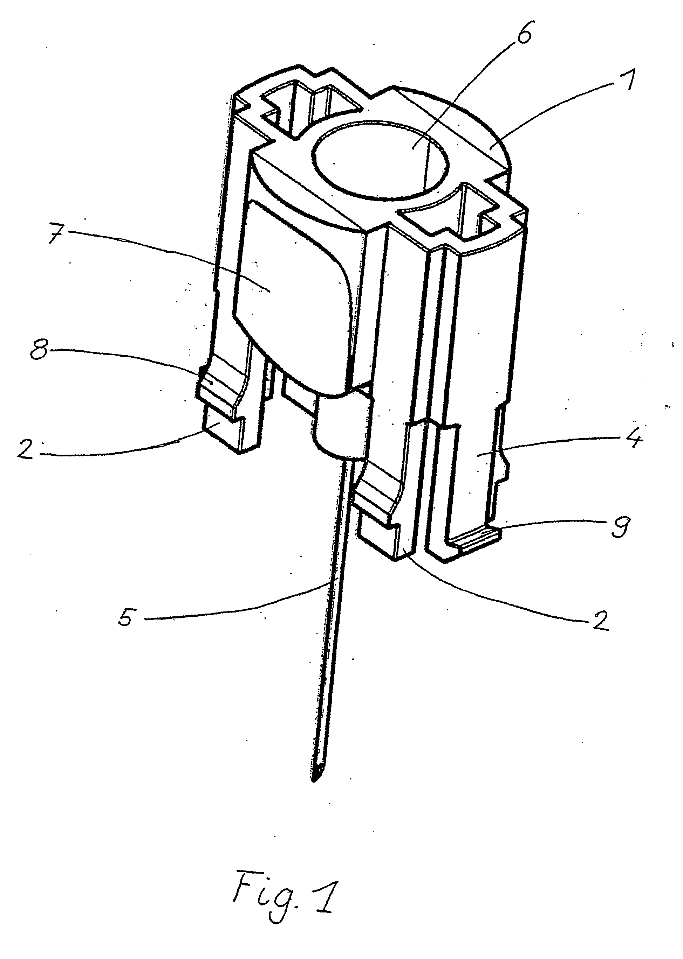

In Figur 1 ist ein Nadelhalter 1 einer Einstechvorrichtung gemäß einer ersten Ausführungsform der Erfindung gezeigt, bei der der Nadelhalter 1 innerhalb einer Schutzvorrichtung (nicht gezeigt) verschiebbar gelagert ist. Eine Injektionsnadel 5 ragt nach unten in Richtung eines Gewebes von dem Nadelhalter 1 ab. Der Nadelhalter 1 weist im wesentlichen eine quaderartige Form auf mit zwei breiten Seiten, von welchen lattenähnliche Fortsätze 2 parallel zur Injektionsnadel 5 abstehen, und zwei kurzen Seiten, von welchen jeweils ein lattenähalicher Fortsatz 4 parallel zur Nadel 5 absteht. Die Fortsätze 2 und 4 bilden einen Teil einer Rasteinrichtung der erfindungsgemäßen Einstechvorrichtung. Der Nadelhalter .1 weist in seinem Inneren eine zylinderartige Öffnung 6 auf, in der weitere Vorrichtungen, wie z. B. eine Nadeleinführvorrichtung, untergebracht werden können. Der Nadelhalter 1 kann jedoch auf der Oberseite auch abgeschlossen sein. An den breiten Seiten des quaderartigen Nadelhalters 1 tritt jeweils eine Grifffläche 7 hervor, an welchen der Nadelhalter 1 von zwei gegenüberliegenden Seiten gefasst und geführt werden kann.

Die Fortsätze 2 der breiten Seite des Nadelhalters .1 ragen derart von dem Nadelhalter 1 ab, dass sie in einem gewissen Maß biegbar sind, d.h. über die von der breiten Seite des Nadelhalters gebildete Ebene hinaus beweglich sind. Der Fortsatz 4 ragt in dieser Ausführungsform steif von dem Nadelhalter 1 an der kurzen Seite ab. Es ist grundsätzlich jedoch auch möglich den Fortsatz 4 biegbar vorzusehen, d.h. er kann über die von der kurzen Seite des Nadelhalters 1 gebildete Ebene hinaus bewegt werden. Durch die Biegeeigenschaft der Fortsätze 2 und gegebenenfalls auch der Fortsätze 4 können die Fortsätze unter Vorspannung versetzt werden. Sie bilden demnach blattfederartige Elemente im Sinne der Erfindung.

An den lattenähnlichen Fortsätzen 2 sind in deren unterem Bereich, d.h. in einem von dem restlichen Nadelhalterkörper entfernt liegenden Bereich, nach außen gerichtete Stufen 8 angeordnet, die eine nach unten gerichtete senkrecht zur Nadelachse verlaufende Fläche aufweisen. An einem unteren Bereich der Fortsätze 4 ist ein bezüglich des Nadelhalters 1 nach außen weisender Vorsprung 9 mit einer nach oben gerichteten senkrecht zur Nadelachse verlaufenden Fläche vorgesehen.

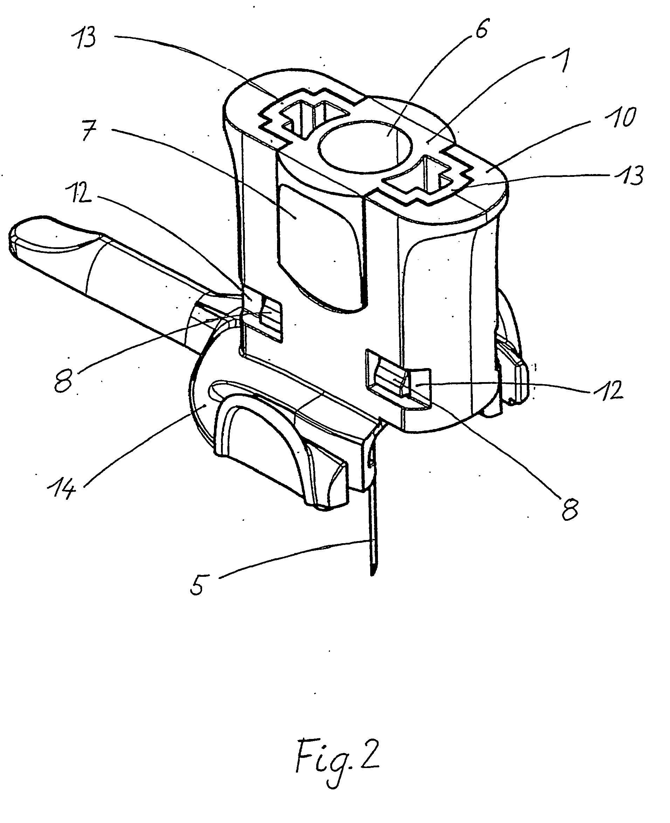

In Figur 2 ist der in Figur 1 gezeigte Nadelhalter 1 in eine Schutzvorrichtung 10 eingesetzt und in einer eingeschobenen Position gezeigt. Die Schutzvorrichtung 10 ist auf einem Grundkörper 14, wie er etwa auf einen Katheterkopf aufgesetzt werden kann, angeordnet, wobei die Injektionsnadel 5 nach unten durch den Grundkörper 14 hervortritt. Die Schutzvorrichtung 10 weist eine ähnliche quaderartige Form auf wie der Nadelhalter 1, und besitzt im Inneren einen Hohlraum passend für den Nadelhalter 1. An einer breiten Seite der Wandung der Schutzvorrichtung 10 ist eine Aussparungen vorgesehen, in welche eine Grifffläche 7 formschlüssig eingefügt werden kann.

In einem unteren Bereich der breiten Seite der. Schutzvorrichtung 10 sind in der Wandung der Schutzvorrichtung 10 Öffnungen 12 angeordnet, in welche die Stufen 8 der lattenähnlichen Fortsätze 2 des Nadelhalters 1 in eingeschobener Position des Nadelhalters eingreifen. In dieser Position befinden sich die lattenähnlichen Fortsätze 2 in einem entspanntem Zustand oder in einem Zustand mit nur geringer Vorspannung, in dem die Fortsätze 2 nach innen gebogen werden.

An der kurzen Seite der quaderartigen Schutzvorrichtung 10 weist die Schutzvorrichtung 10 an ihrer Innenfläche jeweils parallel zur Nadelachse verlaufende Führungsrillen 13 auf. , Die lattenähnlichen Fortsätze 4 des Nadelhalters 1 verlaufen wenigstens teilweise innerhalb der Führungsrillen 13. Jedenfalls ragen die Vorsprünge 9 der Fortsätze 4 im Inneren der Schutzvorrichtung 10 in die Führungsrillen 13 hinein.

Indem die Stufen 8 durch die Öffnungen 12 hervortreten, ergibt sich ein Verriegelungsmechanismus, durch den ein Verschieben des Nadelhalters 1 gegenüber der Schutzvorrichtung 10 erschwert wird. Um den Nadelhalter 1 gegenüber der Schutzvorrichtung 10 verschieben zu können, muss ein erhöhter Anfangswiderstand überwunden werden, der durch an den Stufen 8 angeordnete Abschrägungen, die an einer Kante der Öffnungen 12 der Wandung der Schutzvorrichtung angrenzen, gebildet wird. Die Schutzvorrichtung 10 ist in Richtung der Nadelachse fest mit dem Grundkörper 14 verbunden. Zum Herausziehen des Nadelhalters 1 aus der Schutzvorrichtung 10 muss der erhöhte Anfangswiderstand überwunden werden, wobei die Abschrägungen der Stufen 8 an den Kanten der Öffnungen 12 der Schutzvorrichtung 10 entlang geführt werden, so dass die lattenähnlichen Fortsätze 2 nach innen gebogen und vorgespannt werden bis die Außenseite der Stufen 8 an der Innenseite der Wandung der Schutzvorrichtung zuliegen kommen. Zum Herausziehen des Nadelhalters 1 aus der Schutzvorrichtung 10 ist dann lediglich die Reibungskraft zwischen den Stufen. 8 und der Innenseite der Schutzvorrichtung 10 zu überwinden. Es ist jedoch auch möglich, den Eingriff der Stufen 8 in die Öffnungen 12 zu lösen, indem durch Eindrücken der Griffflächen 7 die Fortsätze 2 geringfügig nach innen gebogen werden. Beim Loslassen des Nadelhalters 1 bewegen sich die Fortsätze 2 wieder nach außen.

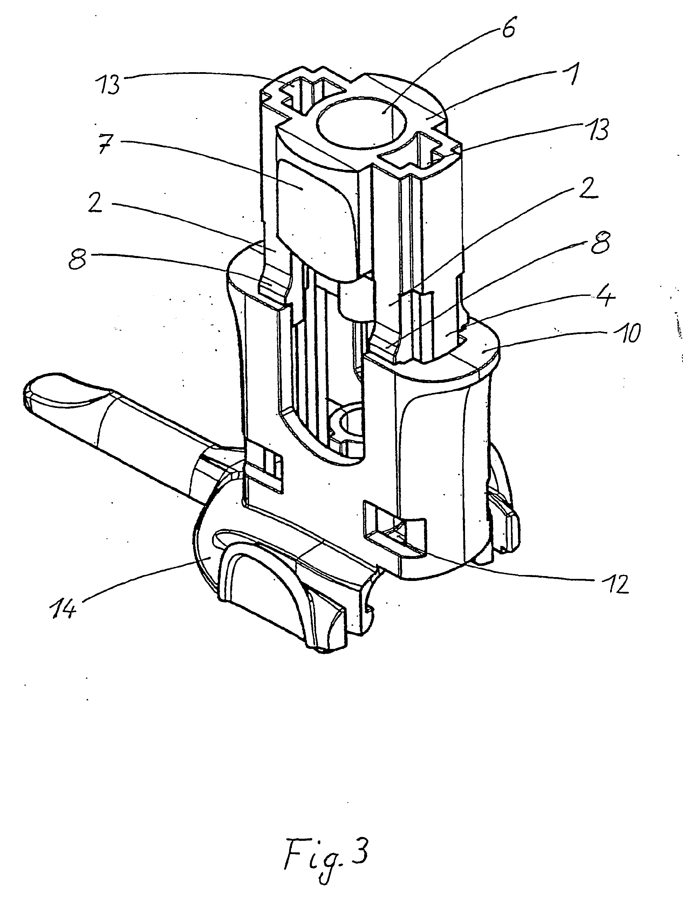

In Figur 3 ist die Einstechvorrichtung nach der ersten Ausführungsform mit einem aus der Schutzvorrichtung 10 herausgezogenen Nadelhalter 1 gezeigt. Um den Nadelhalter 1 aus der eingeschobenen Position in die herausgezogene Position zu verschieben, wird der Nadelhalter 1 an den Griffflächen 7 gefasst und entgegen der Einstechrichtung der Injektionsnadel 5 nach oben aus der Schutzvorrichtung 10 herausgezogen. Der Nadelhalter 1 wird solange aus der Schutzvorrichtung 10 herausgezogen, bis die Vorsprünge 9 der Fortsätze 4 an die Wand der Oberseite der Schutzvorrichtung 10, welche die Führungsrillen 13 begrenzt anstoßen. Gleichzeitig mit dem Anstoßen der Vorsprünge 9 an der Wand der Schutzvorrichtung 10 treten die Stufen 8 der Fortsätze 2 aus der Oberseite der Schutzvorrichtung 10, d.h. über die obere Wand der Schutzvorrichtung, hinaus. Dabei bewegen sich die Fortsätze 2 aufgrund ihrer Vorspannung nach außen und die nach unten gerichtete Fläche der Stufen 8 kommt gegenüber der oberen Wandung der Schutzvorrichtung 10 zuliegen. In dieser Position ist der Nadelhalter in einer gegenüber der Schutzvorrichtung 10 herausgezogenen Position.

Die Schutzvorrichtung 10 kann an der Innenseite ihrer breiten Seite Rastungen z. B. in Form von Stufen oder Vertiefungen aufweisen, welche während dem Herausziehen des Nadelhalters 1 gegenüber den Stufen 8 der Fortsätze 2 zuliegen kommen. Beim Herausziehen des Nadelhalters 1 aus der Schutzvorrichtung 10 können die Stufen 8 der Fortsätze 2 aufgrund ihrer Vorspannung in die Vertiefungen greifen, bzw. oberhalb der Stufen einrasten, so dass der Nadelhalter 1 auch beim Herausziehen aus der Schutzvorrichtung 10 durch diese Rastungen an einem Zurückschieben in die Schutzvorrichtung 10 blockiert wird.

In der gezeigten ersten Ausführungsform bilden die lattenähnlichen Fortsätze 2 und die Stufen 8 mit der Aussenseite der oberen Wandung der Schutzvorrichtung 10 als Widerlager für die Stufen 8 einen ersten Anschlag, der ein Einschieben bzw. Zurückschieben des Nadelhalters 1 in die Schutzvorrichtung 10 hinein blockiert. Die Fortsätze 4 und die Vorsprünge 9 bilden mit der Innenseite der oberen Wandung der Schutzvorrichtung 10, bzw. der Begrenzung der Führungsrillen 13 als Widerlager für die Vorsprünge 9 einen zweiten Anschlag, der ein weiteres Herausziehen des Nadelhalters 1 aus der Schutzvorrichtung 10 blockiert. In dieser Position ist der Nadelhalter 1 mit der Schutzvorrichtung 10 fest verrastet und die Injektionsnadel 5 wird in Umfangsrichtung vollständig von der Schutzvorrichtung 10 umgeben. Die Einstechvorrichtung kann in dieser Position von der Gewebeoberfläche abgenommen werden und ohne das Risiko einer Verletzung an der Injektionsnadel entsorgt werden.

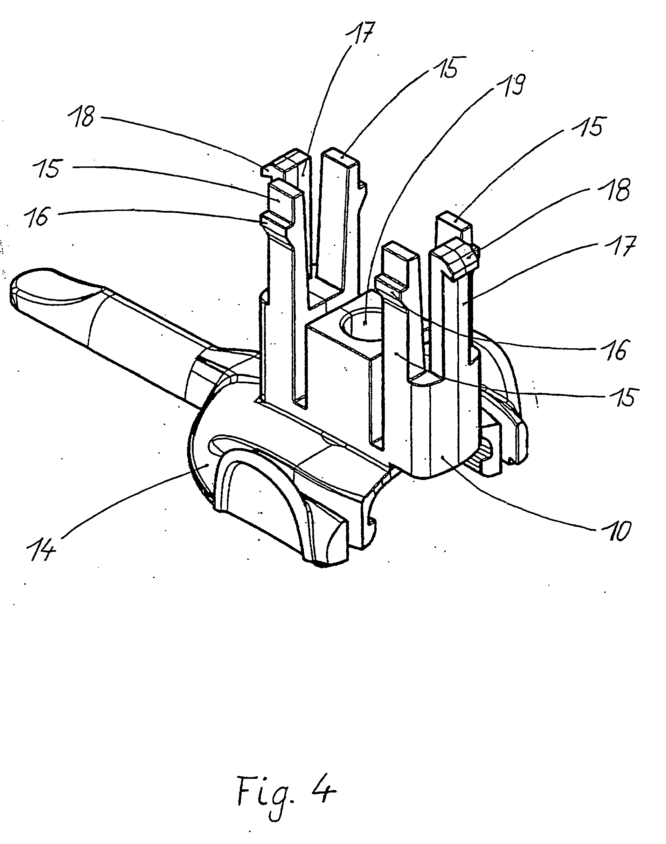

In Figur 4 ist eine Schutzvorrichtung 10 gemäß einer zweiten Ausführungsform einer Einstechvorrichtung nach der vorliegenden Erfindung gezeigt, bei der die Schutzeinrichtung 10 innerhalb des Nadelhalters 1 zu liegen kommt. Die Schutzvorrichtung 10 ist quaderartig mit einer breiten Seite und einer kurzen Seite ausgebildet. An der breiten Seite sind zwei lattenähnliche Fortsätze 15 vorgesehen, die im wesentlichen parallel zur Fläche der breiten Seite verlaufen und nach oben, d.h. in einer Richtung fort von einem Gewebe abstehen. Auf der gegenüberliegenden breiten Seite sind entsprechende Fortsätze 15 ausgebildet. In einem oberen Bereich ist an den Fortsätzen 15 eine Stufe 16 angeordnet, die eine nach oben gerichtete senkrecht zur Nadelachse verlaufende Fläche aufweist und nach außen gerichtet ist. An der kurzen Seite ist ein lattenähnlicher Fortsatz 17 ausgebildet, der im wesentlichen parallel zur Fläche der kurzen Seite verläuft und analog zu den Fortsätzen 15 nach oben von der Schutzvorrichtung 10 absteht. Auf der gegenüberliegenden kurzen Seite der Schutzeinrichtung 10 ist ein entsprechender Fortsatz 17 ausgebildet. Am Ende der Fortsätze 17 ist ein Vorsprung 18 vorgesehen, der von der Schutzvorrichtung 10 nach außen gerichtet ist und eine nach unten weisende senkrecht zur Nadelachse verlaufende Fläche aufweist. Die Fortsätze 15 und 17 stehen derart biegbar von der Schutzvorrichtung 10 ab, dass sie blattfederartig vorspannbar sind, wie die Fortsätze 2 und 4 der ersten Ausführungsform. Die Fortsätze 15 und 17 mit den Stufen 16 und den Vorsprüngen 18 bilden einen Teil der erfindungsgemäßen Rasteinrichtung.

Im mittleren Bereich der Schutzvorrichtung 10 ist eine Öffnung 19 ausgebildet. Eine an dem Nadelhalter 1 angebrachte Injektionsnadel 5, die senkrecht nach unten in Richtung eines Gewebes von dem Nadelhalter 1 absteht, wird durch die Öffnung 19 der Schutzvorrichtung 10 geführt und durchragt auch den Grundkörper 14, so dass sie nach unten aus dem Grundkörper 14 hervorsteht und in ein Gewebe eingestochen werden kann.

Die Schutzvorrichtung 10 der Einstechvorrichtung ist in Figur 4 auf die Oberseite eines Grundkörpers 14 aufgesetzt, der einen Teil eines Katheterkopfes bildet. Der Grundkörper 14 wird mit seiner Unterseite auf die Oberfläche eines Gewebes aufgesetzt, so dass auch die Schutzvorrichtung 10 gegenüber dem Gewebe feststeht.

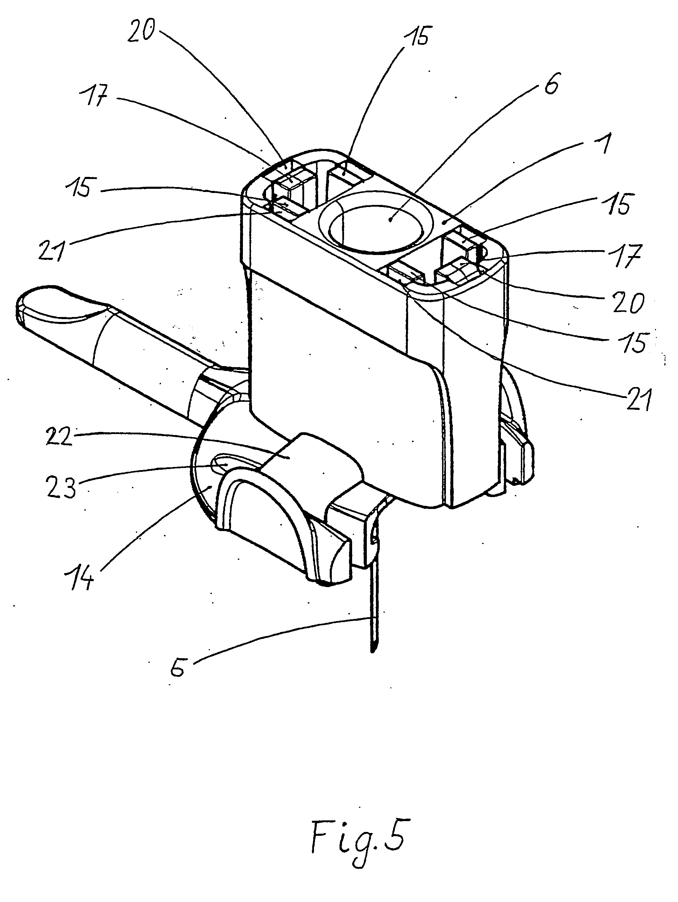

In Figur 5 ist ein Nadelhalter 1 über die Schutzvorrichtung 10 aufgeschoben. Der Nadelhalter 1 befindet sich in einer mit der Schutzvorrichtung 10 zusammengeschobenen Position. Der Nadelhalter 1 ist im wesentlichen derart quaderförmig ausgebildet, dass er schiebbar auf die Schutzvorrichtung 10 passt. Zur Führung des Nadelhalters 1 auf der Schutzvorrichtung 10 sind an den Innenflächen des Nadelhalters 1 Führungsrillen 20 gegenüber den Fortsätzen 17 und Führungsrillen 21 gegenüber den Fortsätzen 15 vorgesehen. In die Führungsrillen 20, bzw. 21, greifen wenigstens die Stufen 16 der Fortsätze 15, bzw. die Vorsprünge 18 der Fortsätze 17, teilweise ein oder es wird auch ein Teil der Fortsätze aufgenommen. Beim Auseinanderziehen des Nadelhalters und der Schutzvorrichtung werden die Stufen 16 und die Vorsprünge 18 innerhalb der Führungsrillen geführt, so dass eine einfache gleitende Bewegung ermöglicht wird Die Führungsrillen 21 für die Fortsätze 15 können eine Art Rampe aufweisen, um beim Auseinanderziehen des Nadelhalters 1 und der Schutzvorrichtung 10 die Fortsätze 15 vorzuspannen. Die Führungsrillen 20 weisen am unteren Ende einen Abschluss parallel zur Unterseite der Schutzvorrichtung 10 auf, so dass beim Auseinanderziehen des Nadelhalters 1 und der Schutzvorrichtung 10 der Vorsprung 18 des Fortsatzes 17 gegen diesen Abschluss stößt. Die Führungsrillen 21 für die Fortsätze 15 sind hingegen nach unten ausder Schutzvorrichtung offen, d.h. sie münden in die Unterseite der Schutzvorrichtung 10. Die Stufen 16 der Fortsätze 15 könnten daher beim Auseinanderziehen aus den Führungsrillen 21 in Richtung der Unterseite des Nadelhalters 1 austreten.

Der Nadelhalter 1 weist an der unteren Kante der breiten Seiten jeweils senkrecht von diesen abstehende Flügel 22 auf, die in ihrem Endbereich nach unten abgewinkelt sind. Mit dem abgewinkelten Teil können die Flügel 22 in Rillen oder Aussparungen 23 des Grundkörpers 14 eingreifen und tragen so zur Stabilisierung des Nadelhalters 1 bei. Es ist auch möglich, durch die Flügel 22 z. B. einen Rastmechanismus oder Verriegelungsmechanismus des Grundkörpers 14 zu blockieren.

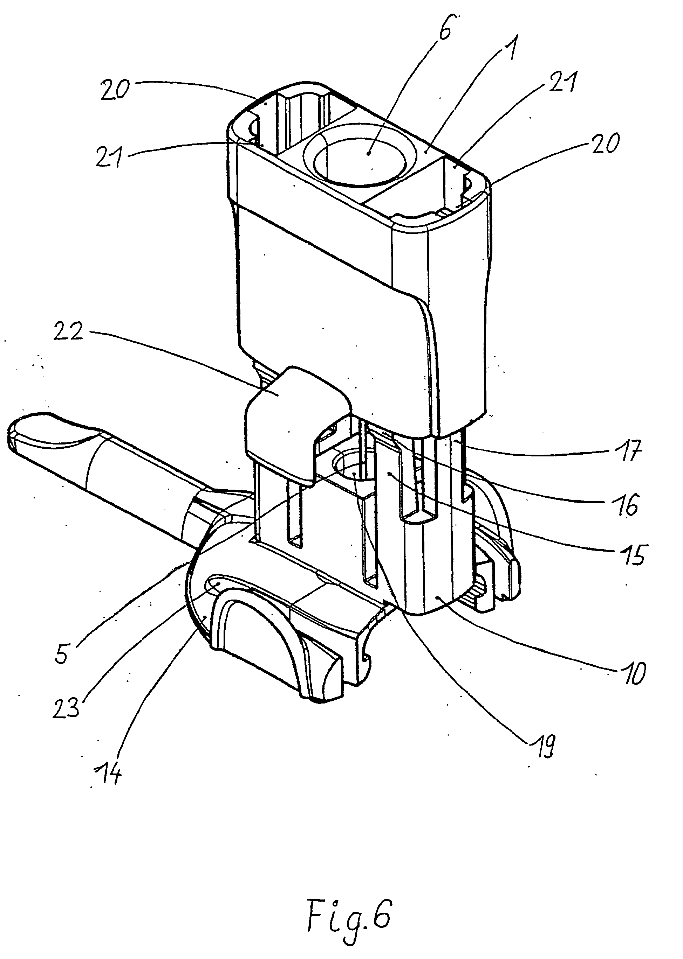

In Figur 6 ist die Einstechvorrichtung nach der zweiten Ausführungsform in einer ausgezogenen Position gezeigt, bei der der Nadelhalter 1 und die Schutzvorrichtung 10 auseinandergezogen sind. Der Nadelhalter 1 kann solange von der Schutzvorrichtung 10 abgezogen werden, bis die Vorsprünge 18 der Fortsätze 17 an den Abschluss der Führungsrillen 20 stoßen. Gleichzeitig treten die Stufen 16 der Fortsätze 15 aus den Führungsrillen 21 aus und greifen durch ihre Vorspannung vor die Unterseite des Nadelhalters 1, d.h. die Unterkante der Wandung der breiten Seite des Nadelhalters 1. In dieser Position ist der Nadelhalter 1 und die Schutzvorrichtung 10 fest miteinander verrastet, da ein weiteres Abziehen des Nadelhalters 1 durch den Anschlag der Fortsätze 17 an den Abschlüssen der Führungsrillen 20 blockiert wird und ein erneutes Zusammenschieben des Nadelhalters 1 über die Schutzvorrichtung 10 durch den Anschlag der Stufen 16 der Fortsätze 15 an den Unterkanten des Nadelhalters 1 blockiert wird.

In dem ausgezogenen Zustand ist die Injektionsnadel 5 vollständig in die Schutzvorrichtung 10 zurückgezogen und wird in Umfangsrichtung von dieser umgeben. Vor allem die Spitze der Injektionsnadel 5 verbleibt innerhalb der Öffnung 19 der Schutzvorrichtung 10, selbst wenn mittlere Bereiche der Injektionsnadel sichtbar werden. Der Block, in dem die Öffnung 19 vorgesehen ist, kann jedoch derart lang ausgebildet werden, dass alle Bereiche der Injektionsnadel von ihm umgeben werden und die Injektionsnadel 5 nicht mehr sichtbar ist.

Wie bei der ersten Ausführungsform der vorliegenden Erfindung ist es auch bei der zweiten Ausführungsform möglich, z. B. in den Führungsrillen 21 für die Fortsätze 15 Rillen oder Stufen vorzusehen, so dass die Stufen 16 beim Abziehen des Nadelhalters 1 von der Schutzvorrichtung 10 bei einem Zurückziehen an einer momentan gegenüberliegenden Rille oder Stufe einschlagen und ein erneutes Aufschieben des Nadelhalters 1 auf die Schutzvorrichtung 10 blockiert wird. Dadurch wird nach einem Einstechen der Injektionsnadel und dem Beginn des Herausziehens der Injektionsnadel aus dem Gewebe durch das Abziehen des Nadelhalters 1 von der Schutzvorrichtung 10 ein erneutes Einstechen der Injektionsnadel in das Gewebe unterbunden. Dient die Injektionsnadel 5 beispielsweise als Einstechvorrichtung bei einem Katheterkopf, wird dadurch eine Beschädigung der flexiblen Kanüle durch die Nadelspitze verhindert.

Die vorliegende Erfindung wurde an einem ersten Ausführungsbeispiel mit an dem Nadelhalter 1 angeordneten lattenähnlichen Fortsätzen 2 und 4 und an einem zweiten Ausführungsbeispiel mit an der Schutzvorrichtung 10 vorgesehenen lattenähnlichen Fortsätzen 15 und 16 dargestellt. Grundsätzlich ist es jedoch auch möglich derartige Fortsätze sowohl an dem Nadelhalter 1 als auch an der Schutzvorrichtung 10 vorzusehen, wobei in dem jeweils komplementären Teil Führungsrillen für die Fortsätze vorgesehen sein können. Ferner ist es auch möglich das Blockieren eines weiteren Herausziehens und das Blockieren eines Zurückschiebens in der ausgezogenen Position von Nadelhalter 1 und Schutzvorrichtung 10 an demselben Fortsatz auszubilden. Die Zahl der lattenähnlichen Fortsätze kann variiert werden, solange ein festes Verrasten des Nadelhalters 1 und der Schutzvorrichtung 10 sichergestellt ist.

Eine Einstechvorrichtung nach der vorliegenden Erfindung kann, wie beschrieben, besonders vorteilhaft als Einführhilfe für eine flexible Kanüle an einem Katheterkopf eingesetzt werden. Grundsätzlich ist es jedoch möglich, eine erfindungsgemäße Einstechvorrichtung auch bei anderen medizinischen und pharmazeutischen Anwendungen zu verwenden. Insbesondere kann ein fluider Wirkstoff auch direkt durch die Injektionsnadel verabreicht werden.

### Bezugszeichen:

- 1: Nadelhalter
- 2: Fortsatz
- 4: Fortsatz
- 5: Injektionsnadel
- 6: Öffnung
- 7: Grifffläche
- 8: Stufe
- 9: Vorsprung
- 10: Schutzvorrichtung
- 11: ---
- 12: Öffnung
- 13: Führungsrille
- 14: Grundkörper
- 15: Fortsatz
- 16: Stufe
- 17: Fortsatz
- 18: Vorsprung
- 19: Öffnung
- 20: Führungsrille
- 21: Führungsrille
- 22: Flügel
- 23: Rille

## Patentansprüche

1. Einstechvorrichtung zum Einstechen einer Injektionsnadel (5) in ein Gewebe, umfassend einen Nadelhalter (1), von dem die Injektionsnadel (5) abragt, und
eine Schutzvorrichtung (10) zum Schutz der Injektionsnadel (5) nach einem Herausziehen aus dem Gewebe,
wobei
der Nadelhalter (1) gegenüber der Schutzvorrichtung (10) entlang der Nadelachse verschiebbar gelagert ist und
zum Einstechen der Injektionsnadel (5) der Nadelhalter (1) in einer mit der Schutzvorrichtung (10) zusammengeschobenen Position ist,
wobei an dem Nadelhalter (1) und/oder der Schutzvorrichtung (10) wenigstens eine Rasteinrichtung vorgesehen ist, die den Nadelhalter (1) und die Schutzvorrichtung (10) wenigstens in einer auseinandergezogenen Position fest miteinander verrastet, wobei
nach einem Auseinanderziehen von Nadelhalter (1) und Schutzvorrichtung (10) ein Zurückschieben des Nadelhalters (1) relativ zu der Schutzvorrichtung (10) blockiert ist, **dadurch gekennzeichnet, dass**;
die Schutzvorrichtung (10) mit einem auf dem Gewebe aufliegenden Grundkörper (14), eines Katheterkopfs derart lösbar verbunden ist, dass die Bewegung zum Lösen der Einstechvorrichtung senkrecht zur Bewegung zum Herausziehen der Injektionsnadel verläuft.

2. Einstechvorrichtung nach Anspruch 1, bei der beim Auseinanderziehen des Nadelhalters (1) und der Schutzvorrichtung (10) die Schutzvorrichtung gegenüber dem Gewebe feststeht.

3. Einstechvorrichtung nach Anspruch 1 oder 2, bei der die Injektionsnadel (5) in der ausgezogenen Position des Nadelhalters (1) in Umfangsrichtung von der Schutzvorrichtung (10) umgeben ist.

4. Einstechvorrichtung nach einem der vorhergehenden Ansprüche, die in der ausgezogenen Position des Nadelhalters (1) in einer Richtung parallel zur Gewebeoberfläche von dem Grundkörper (14) abziehbar ist.

5. Einstechvorrichtung nach einem der vorhergehenden Ansprüche, bei der die Rasteinrichtung wenigstens einen ersten Anschlag zum Blockieren des Nadelhalters (1) in Richtung des Auseinanderziehens und wenigstens einen zweiten Anschlag zum Blockieren des Nadelhalters (1) in Richtung des Zusammenschiebens aufweist.

6. Einstechvorrichtung nach einem der vorhergehenden Ansprüche, bei der die Rasteinrichtung blattfederartige Elemente (2, 4; 15, 17) umfasst.

7. Einstechvorrichtung nach dem vorhergehenden Anspruch, bei der der wenigstens eine erste und/oder der wenigstens eine zweite Anschlag eine Stufe (8; 16), bzw. einen Vorsprung (9; 18) an einem blattfederartigen Element (2, 4; 15, 17) umfasst.

8. Einstechvorrichtung nach einem der vorhergehenden Ansprüche, bei der ein blattfederartiges Element (2, 4; 15, 17) in der zusammengeschobenen Position des Nadelhalters (1) in einer vorgespannten Stellung und in der ausgezogenen Position in einer entspannten Blockierstellung ist.

9. Einstechvorrichtung nach einem der vorhergehenden Ansprüche, bei der ein blattfederartiges Element (2, 4; 15, 17) beim Auseinanderziehen von Nadelhalter (1) und Schutzvorrichtung (10) in eine vorgespannte Stellung bringbar ist und in der ausgezogenen Position in einer entspannten Blockierstellung ist.

10. Einstechvorrichtung nach einem der vorhergehenden Ansprüche, bei der eine Verriegelungsvorrichtung zum Ver- und Entriegeln des Nadelhalters (1) in der zusammengeschobenen Position vorgesehen ist.

11. Katheterkopf für medizinische und pharmazeutische Anwendungen mit einem Grundkörper (14) und einer von diesem abragenden flexiblen Kanüle, der eine Einstechvorrichtung zum Einführen der flexiblen Kanüle in eine Gewebe umfasst, wobei die Einstechvorrichtung nach einem der Ansprüche 1 bis 10 ausgebildet ist.

12. Katheterkopf nach Anspruch 11, bei dem die Injektionsnadel (5) der Einstechvorrichtung eine Einstechhilfe für die flexible Kanüle bildet.

## Claims

1. Puncturing device for inserting an injection needle (5) into a tissue, comprising a needle holder (1), from which the injection needle (5) projects down, and a protection device (10) for the protection of the injection needle (5) after its removal from the tissue,
wherein
the needle holder (1) is movably supported relative to protection device (10) along the needle axis and
to insert the injection needle (5), the needle holder (1) is in a position pushed together with the protection device (10),
wherein there is provided at the needle holder (1) and/or the protection device (10) at least one catching means which firmly locks the needle holder (1) and the protection device (10) together at least in a pulled-apart position, wherein
after needle holder (1) and protection device (10) have been pulled apart a pushing bad movement of the needle holder (1) relative to the protection device (10) is blocked, **characterized in that**:
the protection device (10) is releasably connected to a base (14), resting on the tissue, of a catheter head such that the movement to release the puncturing device is perpendicular to the movement to remove the injection needle.

2. Puncturing device according to claim 1 in which, when the needle holder (1) and the protection device (10) are pulled apart, the protection device remains fixed relative to the tissue.

3. Puncturing device to claim 1 or 2 in which, in the extended position of the needle holder (1), the injection needle (5) is surrounded in peripheral direction by the protection device (10).

4. Puncturing device according to one of the preceding claims which, in the extended position of the needle holder (1), can be pulled off from the base (14) in a direction parallel to the tissue surface.

5. Puncturing device according to one of the preceding claims, in which the catching means has at least one first stop for blocking the needle holder (1) in the direction of the pulling apart and at least one second stop for blocking the needle holder (1) in the direction of the pushing together.

6. Puncturing device according to one of the preceding claims, in which the catching means comprises leaf spring-like elements (2, 4; 15, 17).

7. Puncturing device according to the preceding claim, in which the at least one first and/or at least one second stop comprises a step (8; 16), or a projection (9; 18) on a leaf spring-like element (2, 4; 15, 17), respectively.

8. Puncturing device according to one of the preceding claims in which, in the pushed-together position of the needle holder (1), a leaf spring-like element (2, 4; 15, 17)is in a pre-loaded position and, in the extended position, is in a relaxed blocking position.

9. Puncturing device according to one of the preceding claims in which, when needle holder (1) and protection device (10) are pulled apart, a leaf spring-like element (2, 4; 15, 17) can be brought into a pre-loaded position and, in the extended position, is in a relaxed blocking position.

10. Puncturing device according to one of the preceding claims, in which a locking device is provided for locking and unlocking the needle holder (1) in the pushed-together position.

11. Catheter head for medical and pharmaceutical applications with a base (14) and a flexible cannula projecting therefrom, which comprises a puncturing device for introducing the flexible cannula into a tissue, wherein the puncturing device is formed according to one of the claims 1 to 10.

12. Catheter head according to claim 11, in which the injection needle (5) of the puncturing device forms a puncturing aid for the flexible cannula.

## Revendications

1. Dispositif de piquage pour piquer une aiguille d'injection (5) dans un tissu, comprenant
un porte-aiguille (1) depuis lequel fait saillie l'aiguille d'injection (5), et un dispositif protecteur (10) pour protéger l'aiguille d'injection (5) après extraction hors du tissu,
dans lequel
le porte-aiguille (1) est monté avec possibilité de déplacement le long de l'axe de l'aiguille par rapport au dispositif protecteur (10), et
pour piquer l'aiguille d'injection (5), le porte-aiguille (1) est déplacé dans une position commune avec le dispositif protecteur (10),
sur le porte-aiguille (1) et/ou sur le dispositif protecteur (10) est prévu au moins un dispositif d'enclenchement qui enclenche fermement l'un avec l'autre le porte-aiguille (1) et le dispositif protecteur (10) au moins dans une position en écartement l'un par rapport à l'autre, et
après écartement du porte-aiguille (1) et du dispositif protecteur (10), un retour du porte-aiguille (1) par rapport au dispositif protecteur (10) est bloqué,
**caractérisé en ce que** :
le dispositif protecteur (10) est relié à un corps de base (14) d'une tête de cathéter, lequel est appliqué sur le tissu, la liaison étant libérable de telle façon que le déplacement pour libérer le dispositif de piquage s'étend perpendiculairement au mouvement pour l'extraction de l'aiguille d'injection.

2. Dispositif de piquage selon la revendication 1, dans lequel, lors de l'écartement du porte-aiguille (1) et du dispositif protecteur (10), le dispositif protecteur est immobile par rapport au tissu.

3. Dispositif de piquage selon la revendication 1 ou 2, dans lequel l'aiguille d'injection (3) dans la position en écartement du porte-aiguille (1), est entourée par le dispositif protecteur (10) en direction périphérique.

4. Dispositif de piquage selon l'une des revendications précédentes qui, dans la position en écartement du porte-aiguille (1), est susceptible d'être extrait depuis le corps de base (14) dans une direction parallèle à la surface du tissu.

5. Dispositif de piquage selon l'une des revendications précédentes, dans lequel le dispositif d'enclenchement comprend au moins une première butée pour bloquer le porte-aiguille (1) en direction d'écartement, et au moins une seconde butée pour bloquer le porte-aiguille (1) en direction de la position commune.

6. Dispositif de piquage selon l'une des revendications précédentes, dans lequel le dispositif d'enclenchement comprend des éléments analogues à des ressorts à lames (2, 4 ; 15, 17).

7. Dispositif de piquage selon la revendication précédente, dans lequel ladite au moins une première butée et/ou ladite au moins une seconde butée comprend un gradin (8 ; 16) ou respectivement une saillie (9 ; 18) sur un élément analogue à un ressort à lame (2, 4 ; 15, 17).

8. Dispositif de piquage selon l'une des revendications précédentes, dans lequel un élément analogue à un ressort à lame (2, 4 ; 15, 17) est dans une situation précontrainte dans la position commune du porte-aiguille (1), et est dans une situation de blocage détendue dans la position en écartement.

9. Dispositif de piquage selon l'une des revendications précédentes, dans lequel un élément analogue à un ressort à lame (2, 4 ; 15, 17) est susceptible d'être amené dans une situation précontrainte lors de la mise en écartement du porte-aiguille (1) et du dispositif protecteur (10), et est dans une situation de blocage détendue dans la position en écartement.

10. Dispositif de piquage selon l'une des revendications précédentes, dans lequel est prévu un dispositif de verrouillage pour verrouiller et déverrouiller le porte-aiguille (1) dans la position commune.

11. Tête de cathéter pour des applications médicales et pharmaceutiques, comprenant un corps de base (14) et une canule flexible dépassant de celui-ci, qui englobe un dispositif de piquage pour introduire la canule flexible dans un tissu, le dispositif de piquage étant réalisé selon l'une des revendications 1 à 10.

12. Tête de cathéter selon la revendication 11, dans laquelle l'aiguille d'injection (5) du dispositif de piquage forme une aide au piquage pour la canule flexible.
